# DEMANDE DE BREVET EUROPEEN

(11) **EP 1 155 686 A1**
(43) Date de publication de la demande: **21.11.2001**
(21) Numéro de dépôt: 01401110.0
(22) Date de dépôt: 27.04.2001
(51) Int. Cl.: A61K 7/48

(54) **Utilisation du maganèse dans le traitement des rides**

(30) Priorité: 18.05.2000 FR 0006373
(71) Demandeur: L'OREAL, 75008 Paris (FR)
(72) Inventeur: Nonotte, Isabelle, 75017 Paris (FR); Breton, Lionel, 78000 Versailles (FR)
(74) Mandataire: Galup, Cédric Olivier Nicolas

(57) **Abrégé**

La présente invention se rapporte à l'utilisation d'une quantité efficace du manganèse ou de ses sels dans une composition ou pour la préparation d'une composition, le manganèse ou la composition étant destinés à relâcher et/ou relaxer le tissu cutané et/ou sous-cutané, notamment en vue de traiter les rides et les ridules de la peau.

## Description

La présente invention se rapporte à l'utilisation d'une quantité efficace du manganèse ou de ses sels dans une composition ou pour la préparation d'une composition, le manganèse ou la composition étant destinés à relâcher et/ou relaxer le tissu cutané et/ou sous-cutané, notamment en vue de traiter les rides et les ridules de la peau.

Les femmes, voire même les hommes, ont tendance actuellement à vouloir paraître jeunes le plus longtemps possible et cherchent par conséquent à estomper les marques du vieillissement de la peau, qui se traduisent notamment par des rides et des ridules. A ce sujet, la publicité et la mode font état de produits destinés à garder le plus longtemps possible une peau éclatante et sans ride, marques d'une peau jeune, d'autant plus que l'aspect physique agit sur le psychisme et/ou sur le moral.

Jusqu'à présent, on traitait les rides et les ridules à l'aide de produits cosmétiques contenant des actifs agissant sur la peau, par exemple en l'hydratant ou en améliorant son renouvellement cellulaire ou encore en favorisant la synthèse du collagène qui compose le tissu cutané. Mais, à ce jour on ne sait pas agir sur les rides en intervenant sur les éléments contractiles présents dans la peau.

Ainsi, il est connu que les muscles peauciers du visage sont sous le contrôle des afférences nerveuses motrices du nerf facial et que, par ailleurs, les cloisons interlobulaires de l'hypoderme contiennent en leur sein des fibres qui constituent un tissu musculaire strié (panniculus carnosus). D'autre part, il est également connu qu'une sous-population de fibroblastes du derme, que l'on appelle myofibroblastes, présente des caractéristiques contractiles communes avec le tissu musculaire.

Le calcium est le messager final de la contraction musculaire. Le cycle contraction-relâchement est dû aux variations de la concentration du calcium cytoplasmique de 10⁻⁸ à 10⁻⁵ M dans la cellule contractile.

Dans le muscle au repos, la concentration intracellulaire du calcium libre reste inférieure à 10⁻⁸ M bien que la concentration extracellulaire soit 10.000 fois plus élevée et que la force représentée par le gradient de potentiel électrochimique tende à faire pénétrer le calcium dans la cellule. Cet état de repos est dû à la faible perméabilité de la membrane cellulaire pour le calcium et à l'activité de divers mécanismes qui séquestrent le calcium ou l'expulsent de la cellule.

Diverses protéines cytoplasmiques, notamment les parvalbumines ont ainsi la capacité de lier le calcium. Parmi les organelles intracellulaires, le réticulum endoplasmique peut accumuler et relarguer le calcium dans des conditions compatibles avec une régulation physiologique.

L'élévation du taux de calcium dans le cytoplasme de la cellule musculaire permet l'activation de la machinerie contractile. L'entrée du calcium dans le compartiment intracellulaire (dépolarisation) participe à la diminution de la différence de potentiel entre l'extérieur et l'intérieur et rend ainsi la cellule plus excitable.

En effet, la dépolarisation des tubules transverses (invagination de la membrane cellulaire) qui se propage aux tubules longitudinaux (reticulum sarcoplasmique) induit la libération momentanée du calcium intracellulaire par ces dernières. En présence de calcium les protéines contractiles du muscle strié présentent une activité ATPase qui fournit l'énergie nécessaire à la contraction.

A l'inverse, le relâchement du muscle strié apparaît à la suite de la fixation d'ATP sur les protéines contractiles. Le calcium intracellulaire réintègre alors le compartiment intracellulaire et sa concentration redevient proche d'une valeur de 10⁻⁸M.

Par ailleurs, il a été montré que la toxine botulique, utilisée à l'origine pour traiter les spasmes, pouvait agir sur les états de spasticité musculaire (voir A. Blitzer et al., Arch. Otolaryngol. Head Neck Surg., 1993, 119, pages 1018 à 1022) et sur les rides de la glabelle qui sont les rides inter sourcilières (voir J.D. Carruters et al., J. Dermatol. Surg. Oncol., 1992, 18, pages 17 à 21). En conséquence, il est possible d'agir sur la composante contractile musculaire des rides (notamment au niveau de la plaque motrice qui correspond à la jonction nerf-muscle).

Dans le système nerveux périphérique, la jonction entre un nerf et un muscle constitue la plaque neuro-musculaire, en amont de laquelle se trouve la voie nerveuse efférente appelée motoneurone. Par ailleurs, les membranes cellulaires de chaque fibre nerveuse comportent également de nombreux canaux ioniques, et notamment des canaux calciques, aptes à laisser traverser l'élément correspondant sous forme ionique, dans ce cas particulier le calcium.

On comprend ainsi le rôle important du calcium et de la régulation de sa concentration intracellulaire dans les phénomènes de contraction/relâchement musculaires que ces phénomènes soient pré ou post-jonction cellule nerveuse / cellule non nerveuse (myocytes, myofibroblastes ...).

La régulation de la concentration intracellulaire du calcium n'est possible que parce que l'efflux de calcium corrige l'influx. Ceci ne peut être assuré que par une expulsion du calcium cellulaire par un ou des mécanismes aptes à surmonter le gradient de potentiel électrochimique évoqué plus haut.
Deux types de mécanismes peuvent intervenir : une pompe à calcium qui expulse activement les cations aux dépens de l'hydrolyse d'ATP et un mouvement de calcium passif à travers différents canaux (dépendant du gradient calcique intra et extracellulaire). Dans la plupart des cellules, la pompe à calcium ATP-dépendante opère plus efficacement en présence de la calmoduline qui augmente son affinité.

Afin de mieux décrire les changements de perméabilité au calcium, il est actuellement habituel de considérer que cette perméabilité correspond à l'ouverture de canaux calcium membranaires, canaux opérés par des variations du potentiel de la membrane (VOC) ou l'activation des récepteurs membranaires (ROC). A ce jour, six types VOC de canaux calciques (L, N, T, P, Q, R) ont été identifiés.

On comprend donc de ce qui précède que la contraction ou l'hypercontraction de certains muscles de la face résulte en l'apparition de rides. Cette activation musculaire est elle-même en partie induite par une variation de flux de calcium au travers des canaux calciques transmembranaires.

Or la demanderesse a maintenant découvert après de nombreux tests cliniques, que la fibre musculaire contractile, qui se trouve sous le contrôle direct de l'influx neuro-moteur, joue un rôle essentiel dans la formation des rides et que la modulation de l'influx neuro-moteur et le contrôle de la contraction des fibres musculaires, jouent un rôle essentiel dans la formation des rides. Ainsi la modulation de la contraction motrice atténue non seulement les rides mais également les ridules et présente aussi un effet de « lissage » sur le microrelief cutané. Elle a aussi trouvé que les tissus cutané et sous-cutané comportent des canaux calcium, ce qui, jusqu'à présent, n'a pas été envisagé.

Ainsi, la demanderesse propose d'agir sur les canaux calciques des tissus cutané et sous-cutané pour relâcher ou relaxer les tissus, et ainsi diminuer les rides et les ridules.

Dès 1965, des travaux ont été menés par T. Godfraind afin de rechercher les mécanismes par lesquels certaines substances médicamenteuses inhibaient la réponse contractile à plusieurs agents vasoactifs. L'hypothèse proposée était que la perméabilité de la membrane au calcium pourrait être inhibée par des agents pharmacologiques, ce qui constituerait le mécanisme commun sur lequel agiraient des antagonistes polyvalents.
La technique expérimentale la plus simple, permettant de montrer qu'un agent pharmacologique est à même d'inhiber l'entrée du calcium, consiste à préincuber un muscle lisse dans une solution physiologique dépourvue de calcium, de la dépolariser dans une solution riche en KCI et d'augmenter graduellement la concentration de calcium dans la solution de perfusion. Ceci provoque une augmentation de tension du muscle dont la valeur évolue jusqu'à un maximum en fonction de la concentration de calcium. Lorsque ce protocole est répété en présence d'une substance supposée inhiber l'entrée de calcium ainsi que cela fut réalisé pour la première fois avec la cinnarizine, les réponses contractiles sont inhibées d'une manière dose-dépendante. Un concept semblable a été appliqué pour décrire l'action du vérapamil sur le coeur. Le vérapamil a été d'abord considéré comme un β-bloquant, son action est plus complexe puisqu'il exerce une action inhibitrice sur le couplage excitation-contraction. Sur le muscle papillaire, le vérapamil abolit la contraction en modifiant très faiblement le potentiel d'action. C'est cette observation qui conduit à considérer le vérapamil comme un antagoniste du calcium.

Le manganèse est un métal très répandu à la surface de la croûte terrestre.
Il appartient au groupe Vlla de Mendeleïev, son numéro atomique est 25, son poids atomique est de 54,93. Le manganèse présente plusieurs valences (1 à 7), les formes di- et trivalentes sont celles qui sont biologiquement les plus actives.

Le manganèse est très utilisé dans l'industrie métallurgique, dans la fabrication de piles sèches et comme colorant.

Les végétaux sont tous riches en Mn : particulièrement les graines (environ 7 µg/g), les noix (environ 17 µg/g) et le thé. Les fruits (environ 1 µg/g) et les légumes (environ 2,5 µg/g) sont moins riches, mais leur taux est encore très élevé par rapport aux aliments d'origine animale (viandes : environ 0,20 µg/g, poissons : environ 0,05 µg/g).

A l'inverse chez les animaux, particulièrement l'homme, ce métal ne se trouve qu'à l'état de trace.

Son rôle biologique est cependant très important et même, si les effets néfastes d'une carence n'ont pas été constatés de façon irréfutable chez l'Homme, les conséquences des déficits examinées chez l'animal montrent que ce métal est impliqué dans de nombreux métabolismes. Mais, encore aujourd'hui, les connaissances sur les mécanismes biochimiques intimes de ce métal restent très fragmentaires.
Le manganèse a été impliqué dans de nombreux métabolismes
- la coagulation ;
- la thermogenèse (par son action sur le système thyroïdien)
- l'immunité, où le manganèse semble être nécessaire à une synthèse correcte des anticorps ;
- la reproduction, sa carence entraînant une baisse de fertilité des femelles, et des mâles, peut-être à cause de l'action limitante du manganèse sur la synthèse du cholestérol et de celle des précurseurs des hormones sexuelles.

Deux propriétés permettent d'expliquer une partie du rôle physiopathologique du manganèse :
- l'activation de nombreuses enzymes.
   Le manganèse est un métal qui active de nombreuses enzymes et lectines. Il intervient soit comme un élément dissociable, soit en faisant partie intégrante de la structure de l'enzyme (métalloenzymes).
- son activité inhibitrice vis-à-vis des canaux calcium.
   Le signal interne d'activation d'une cellule est souvent déclenché par une modification des concentrations intracytoplasmiques du calcium. Ce dernier se fixe sur des protéines (calmoduline), qui vont à leur tour activer des kinases.
   Le calcium sert à la transmission de l'influx nerveux, à la stimulations de certaines cellules sécrétantes, il déclenche les changements de forme de la plaquette au début de son activation, etc.

Le manganèse bloque la pénétration du calcium vers le cytoplasme dans de nombreuses cellules à activité sécrétrice (pancréas par exemple), ou électrique ; il inhibe notamment la sortie des neurotransmetteurs au niveau de la plaque motrice. Le manganèse a une action inhibitrice sur la stimulation des lymphocytes B et T, s'il est ajouté au milieu, très peu de temps après le mitogène.

Pour qu'une substance soit reconnue comme un inhibiteur des canaux calcium, autrement appelé dans le texte antagoniste calcique, elle doit pouvoir diminuer la concentration intracellulaire en calcium ou diminuer la liaison du calcium aux protéines intracellulaires comme par exemple la calmoduline, tel que cela est notamment décrit par exemple, par Galizzi, J.P et *al,* J. Biol. Chem. 1987, 262 p 6947 ou Y. Okamiya et *al,* Eur. J. Pharmacol. 1991, 205, p 49 ou J.A. Wagner et al, J. Neurosci. 1988, 8, p 3354 ou H.R Lee et *al*, Life Sci. 1984, 35 p 721 ou Schoemaker H. et Lauger S. Eur. J. Pharmacol. 1985, 111 p 273 ou encore I.J. Reynolds et *al*, J. Pharmacol. Exp. Ther. 1986, 237 p 731.

Une substance est reconnue comme relaxante au sens de l'invention lorsqu'elle montre un effet de relaxation sur un tissu musculaire contracté et/ou montrer un effet inhibiteur dans un modèle expérimental de jonction nerf-muscle (plaque motrice) notamment dans le modèle décrit par W. Steinbrecher dans : Electrodes for stimulation and bioelectric potentiel recording, Ed. Biomerstechnich, 1988, pages 96-98.

Le manganèse ou ses sels répond bien à ces définitions.

Comme précédemment indiqué, la demanderesse propose d'agir sur les canaux calciques pour relâcher ou relaxer les tissus, et ainsi diminuer les rides et les ridules. Pour cela, elle propose l'utilisation du manganèse qu'il soit sous la forme ionique ou sous la forme de sel ou sous la forme d'extraits naturels, végétaux ou de micro-organismes, particulièrement bactériens, riches en manganèse.

Ainsi, la présente invention se rapporte à l'utilisation dans une composition ou pour la préparation d'une composition, dans un milieu physiologiquement acceptable, d'une quantité efficace du manganèse ou d'au moins l'un de ses sels, le manganèse ou la composition étant destinés à relaxer et/ou relâcher le tissu cutané et/ou sous-cutané.

L'invention se rapporte également à l'utilisation dans une composition ou pour la préparation d'une composition, dans un milieu physiologiquement acceptable, d'une quantité efficace d'extraits naturels, végétaux ou de micro-organismes, particulièrement bactériens, riches en manganèse ou en sel de manganèse, l'extrait ou la composition étant destinés à relaxer et/ou relâcher le tissu cutané et/ou sous-cutané.

Par sels de manganèse on entend selon l'invention les sels organiques ou inorganiques du manganèse.

Comme sels organiques utilisables selon l'invention on peut citer le gluconate de manganèse ou le carbonate de manganèse ou l'acétate de manganèse ou le citrate de manganèse ou l'oléate de manganèse ou l'oxalate de manganèse.

Comme sels inorganiques de manganèse on peut citer les sels minéraux comme le chlorure de manganèse ou le borate de manganèse ou le nitrate de manganèse ou le phosphate de manganèse ou le sulfate de manganèse.

Par ailleurs dans le texte, sous réserve d'indication contraire, l'emploi du terme manganèse doit être compris comme signifiant aussi bien le manganèse sous forme ionique, sous forme de sels ou sous forme d'extraits naturels, végétaux ou de micro-organismes, particulièrement bactériens, riches en manganèse.

Par milieu physiologiquement acceptable, on entend compatible avec la peau, le cuir chevelu et/ou les muqueuses.

Un autre objet de l'invention est l'utilisation, dans une composition ou pour la préparation d'une composition, dans un milieu physiologiquement acceptable, d'une quantité efficace du manganèse, le manganèse ou la composition étant destiné à lisser la peau.

Un autre objet encore de l'invention est l'utilisation, dans une composition ou pour la préparation d'une composition, dans un milieu physiologiquement acceptable, d'une quantité efficace du manganèse, le manganèse ou la composition étant destiné à atténuer et/ou effacer le micro-relief de la peau.

Particulièrement, l'invention a pour objet, l'utilisation, dans une composition ou pour la préparation d'une composition, dans un milieu physiologiquement acceptable, d'une quantité efficace du manganèse, le manganèse ou la composition étant destinés à combattre, de manière curative et/ou préventive, les rides et les ridules de la peau.

Cette utilisation s'avère particulièrement efficace pour diminuer les rides et ridules.

Plus particulièrement la relaxation et/ou le relâchement du tissu cutané et/ou sous-cutané correspond à un relâchement ou une relaxation musculaire.

La quantité efficace de manganèse utilisable selon l'invention est bien entendu fonction de l'effet recherché et peut donc varier dans une large mesure.

Pour donner un ordre de grandeur, il est possible d'utiliser selon l'invention du manganèse en une quantité représentant de 0,0001 % à 10% du poids total de la composition et préférentiellement en une quantité représentant de 0,001% à 5% du poids total de la composition.

Bien entendu lorsque selon l'invention on utilise un extrait naturel, végétal ou de micro-organismes, particulièrement bactériens, riche en manganèse, l'homme métier sait adapter la quantité d'extrait à utiliser pour, au final, utiliser le manganèse dans les quantités ci-dessus exposées.

Comme extraits naturels riche en manganèse utilisables selon l'invention, on peut citer les extraits de noix ou les extraits de thé.

Les compositions de l'invention sont destinées à des applications cosmétiques ou dermatologiques. Préférentiellement, les compositions de l'invention sont destinées à des applications cosmétiques.

Les utilisations selon l'invention sont cosmétiques car elles visent à modifier l'aspect de la personne.

Les compositions selon l'invention peuvent se présenter sous toutes les formes galéniques normalement utilisées pour une application topique, injectable ou orale.

La composition selon l'invention peut être appliquée soit par voie locale, c'est-à-dire par voie topique, ou par injection sous-cutanée et/ou intradermique, ou encore par voie orale.

Préférentiellement selon l'invention, la composition est appliquée par voie topique.

Les quantités des différents constituants des compositions selon l'invention sont celles classiquement utilisées dans les domaines considérés et sont appropriées à leur forme galénique.
Pour une application topique, les compositions de l'invention comprennent un milieu compatible avec la peau. Ces compositions peuvent se présenter notamment sous forme de solutions aqueuses, alcooliques ou hydroalcooliques, de gels, d'émulsions eau-dans-huile ou huile-dans-eau ayant l'aspect d'une crème ou d'un gel, de microémulsions, d'aérosols, ou encore sous forme de dispersions vésiculaires contenant des lipides ioniques et/ou non ioniques. Ces formes galéniques sont préparées selon les méthodes usuelles des domaines considérés.

Ces compositions à application topique peuvent constituer notamment une composition de protection, de soin pour le visage, pour le cou, pour les mains ou pour le corps, (par exemple crèmes de jour, crèmes de nuit, crèmes ou huiles solaires, laits corporels), une composition de maquillage (par exemple fond de teint) ou une composition de bronzage artificiel.

Quand la composition de l'invention est une émulsion, la proportion de corps gras qu'elle contient peut aller de 5 % à 80 % en poids, et de préférence de 5 % à 50 % en poids par rapport au poids total de la composition. Les corps gras et les émulsionnants utilisés dans la composition sous forme d'émulsion sont choisis parmi ceux classiquement utilisés dans le domaine cosmétique ou pharmaceutique.

Comme corps gras utilisables dans l'invention, on peut citer les huiles minérales (vaseline), les huiles végétales (fraction liquide de beurre de karité) et leurs dérivés hydrogénés, les . huiles animales, les huiles de synthèse (perhydrosqualène), les huiles siliconées (diméthylpolysiloxane) et les huiles fluorées. Comme autres corps gras, on peut encore citer les alcools gras (alcool cétylique, alcool stéarylique), les acides gras (acide stéarique) et les cires.

Les émulsionnants peuvent être présents, dans la composition, en une proportion allant de 0,3 % à 30 % en poids, et de préférence de 0,5 à 30 % en poids par rapport au poids total de la composition.

De façon connue, la composition de l'invention peut contenir également des adjuvants habituels dans les domaines correspondants, tels que les gélifiants hydrophiles ou lipophiles, les conservateurs, les antioxydants, les solvants, les parfums, les charges, les filtres et les matières colorantes. Par ailleurs, ces compositions peuvent contenir des actifs hydrophiles ou lipophiles. Les quantités de ces différents adjuvants ou actifs sont celles classiquement utilisées dans le domaine cosmétique ou pharmaceutique, et par exemple de 0,01 % à 20 % du poids total de la composition. Ces adjuvants ou ces actifs, selon leur nature, peuvent être introduits dans la phase grasse, dans la phase aqueuse et/ou dans des vésicules lipidiques.

Parmi les actifs que peuvent contenir les compositions de l'invention, on peut notamment citer les actifs ayant un effet sur le traitement des rides ou des ridules, autres que le manganèse, et en particulier les actifs kératolytiques. Par kératolytique, on entend un actif ayant des propriétés desquamantes, exfoliantes ou gommantes, ou un actif capable de ramollir la couche cornée.

Parmi les actifs ayant un effet sur le traitement des rides ou des ridules que peuvent contenir les compositions de l'invention, on peut citer par exemple l'alvérine, les ouvreurs de canaux chlore les hydroxyacides et les rétinoïdes.

Les hydroxyacides peuvent être par exemple des α-hydroxy-acides ou des β-hydroxy-acides, qui peuvent être linéaires, ramifiés ou cycliques, saturés ou insaturés. Les atomes d'hydrogène de la chaîne carbonée peuvent, en outre, être substitués par des halogènes, des radicaux halogénés, alkylés, acylés, acyloxylés, alcoxy carbonylés ou alcoxylés ayant de 2 à 18 atomes de carbone.

Les hydroxyacides qui peuvent être utilisés sont notamment les acides glycolique, lactique, malique, tartrique, citrique, hydroxy-2 alcanoïque, mandélique, salicylique, ainsi que leurs dérivés alkylés comme l'acide n-octanoyl-5-salicylique, l'acide n-dodécanoyl-5-salicylique, l'acide n-décanoyl-5-salicylique, l'acide n-octyl-5-salicylique, l'acide n-heptyloxy-5 ou -4-salicylique, l'acide 2-hydroxy-3-méthylbenzoïque, ou encore leurs dérivés alcoxylés comme l'acide 2-hydroxy-3-méthoxybenzoïque.

Les rétinoïdes peuvent être notamment l'acide rétinoïque (all-trans ou 13-cis) et ses dérivés, le rétinol (vitamine A) et ses esters tels que le palmitate de rétinol, l'acétate de rétinol et le propionate de rétinol, ainsi que leurs sels.

Ces actifs peuvent être utilisés en particulier à des concentrations allant de 0,0001% à 5% en poids par rapport au poids total de la composition.

L'invention a aussi pour objet un procédé de traitement cosmétique des rides et/ou des ridules, consistant à appliquer sur la peau une composition cosmétique comprenant dans un milieu physiologiquement acceptable, une quantité efficace du manganèse.

Le procédé de traitement cosmétique de l'invention peut être mis en oeuvre notamment en appliquant la composition cosmétique telle que définie ci-dessus, selon la technique d'utilisation habituelle de ces compositions. Par exemple : application de crèmes, de gels, de sérums, de lotions, de laits de démaquillage ou de compositions anti-solaires sur la peau ou de compositions pour spray.

Les exemples et compositions suivants illustrent l'invention sans la limiter aucunement. Dans les compositions les proportions indiquées sont des pourcentages en poids, sauf mention contraire.

### Exemple 1 :

Activité du gluconate de manganèse et du chlorure de manganèse dans un modèle de jonction nerf / muscle (plaque motrice) obtenu dans une préparation nerf phrénique / diaphragme isolé : recherche d'un effet myorelaxant :

Le nerf phrénique et le diaphragme sont soigneusement isolés et placés dans une cuve de 50 ml remplie de liquide de survie (liquide de Krebs Henseleit) maintenu à une température de 37°C et oxygéné à l'aide d'un mélange d'oxygène 95 % et de CO₂ 5 %.

Les variations de tension du diaphragme sont ensuite enregistrées avec une précharge initiale de plusieurs grammes.

Après une période de relaxation de 30 mn, le diaphragme est stimulé indirectement par l'intermédiaire du nerf phrénique.

Sur chaque préparation, l'effet des produits à tester a été dans un premier temps, évalué sur les contractions induites par stimulation indirecte *via* stimulation sur le nerf phrénique (0.1 à 7 volts, 0.3 ms, 0.1 Hz) aux concentrations croissantes et cumulées de 10⁻⁶ M à 10⁻³ M.

Résultats obtenus dans le modèle de plaque motrice avec les 2 sels de manganèse.

| Produit | Concentration | % d'inhibition (stimulation indirecte) |
|---|---|---|
| Gluconate de manganèse (n=2) | 10⁻³ M | 25% |
| MnCl₂ | 10⁻³ M | 10% |

### Exemple 2 : exemples de compositions selon l'invention.

| Composition 1 : Lotion de soin antirides pour le visage | |
|---|---|
| Gluconate de manganèse | 1,50 % |
| Antioxydant | 0,05 % |
| Conservateur | 0,30 % |
| Ethanol (solvant) | 8,00 % |
| Eau | qsp 100 % |

La lotion obtenue agit sur les rides lors d'une utilisation répétée (application biquotidienne pendant un mois).

| Composition 2 : Gel pour le soin du visage | |
|---|---|
| Chlorure de manganèse | 0,50% |
| Hydroxypropylcellulose * | 1,00 % |
| Conservateur | 0,30 % |
| Ethanol (solvant) | 15,00 % |
| Antioxydant | 0,05 % |
| Eau | qsp 100 % |

| | |
|---|---|
| * : Klucel H vendu par la société Hercules (gélifiant). | |

Le gel obtenu agit sur les rides. Il peut être appliqué quotidiennement matin et soir pendant un mois.

| Composition 3 : Crème de soin du visage (émulsion huile-dans-eau) | |
|---|---|
| Gluconate de manganèse | 0,50% |
| Stéarate de glycérol (émulsionnant) | 2,00 % |
| Polysorbate 60 (Tween 60 vendu par la société ICI) (émulsionnant) | 1,00 % |
| Acide stéarique | 1,40 % |
| Triéthanolamine (neutralisant) | 0,70 % |
| Carbomer (Carbopol 940 vendu par la société Goodrich) | 0,40 % |
| Fraction liquide de beurre de karité | 12,00 % |
| Perhydrosqualène | 12,00 % |
| Conservateur | 0,30 % |
| Parfum | 0,50 % |
| Antioxydant | qsp 0,05 % |
| Eau | 100 % |

On obtient une crème blanche, onctueuse, qui agit sur les rides et les ridules, et que l'on peut appliquer quotidiennement.

| Composition 4 : Crème de soin du visage (émulsion huile-dans-eau) | |
|---|---|
| Gluconate de manganèse | 0,10 % |
| Mono-, distéarate de glycérol | 2,00 % |
| Alcool cétylique | 1,50 % |
| Mélange alcool cétylstéarylique/alcool cétylstéarylique oxyéthyléné 33 OE | 7,00 % |
| Diméthylpolysiloxane | 1,50 % |
| Huile de vaseline | 17,50 % |
| Conservateur | 0,30 % |
| Parfum | 0,50 % |
| Glycérine | 12,50 % |
| Eau | qsp 100 % |

| Composition 5 : crème de soin du visage (émulsion huile dans eau) | |
|---|---|
| Extrait de noix | 5,00 % |
| Mono, distearate de glycerol | 2,00 % |
| Alcool cetylique | 1,50 % |
| Mélange alcool cétyl stearylique / alcool | |
| Cétyl stearylique oxyethylene 330^{E} | 7,00 % |
| Dimethylpolysiloxane | 1,50 % |
| Huile de vaseline | 17,50 % |
| Conservateur | 0,30 % |
| Parfum | 0,50 % |
| Glycerine | 12,50 % |
| Eau | QSP 100 % |

## Revendications

1. Utilisation dans une composition ou pour la préparation d'une composition d'une quantité efficace de manganèse ou d'au moins l'un de ses sels, le manganèse ou la composition étant destiné à relaxer et/ou relâcher le tissu cutané et/ou sous-cutané.

2. Utilisation dans une composition ou pour la préparation d'une composition d'une quantité efficace de manganèse ou d'au moins l'un de ses sels, le manganèse ou la composition étant destiné à traiter, de manière curative et/ou préventive, les rides et les ridules de la peau.

3. Utilisation selon la revendication précédente, pour diminuer les rides et ridules.

4. Utilisation dans une composition ou pour la préparation d'une composition d'une quantité efficace de manganèse ou d'au moins l'un de ses sels, le manganèse ou la composition étant destiné à lisser la peau.

5. Utilisation dans une composition ou pour la préparation d'une composition d'une quantité efficace de manganèse ou d'au moins l'un de ses sels, le manganèse ou la composition étant destiné à atténuer et/ou effacer le micro-relief de la peau.

6. Utilisation selon la revendication 1, **caractérisée en ce que** la relaxation et/ou le relâchement du tissu cutané et/ou sous-cutané est un relâchement ou une relaxation musculaire.

7. Utilisation selon l'une quelconque des revendications précédentes, **caractérisée en ce que** le manganèse est utilisé en une quantité représentant de 0,0001% à 10% du poids total de la composition.

8. Utilisation selon l'une quelconque des revendications précédentes, **caractérisée en ce que** le manganèse est utilisé en une quantité représentant de 0,001 % à 5 % du poids total de la composition.

9. Utilisation selon l'une quelconque des revendications précédentes, **caractérisée en ce que** le manganèse est sous forme ionique ou sous la forme d'un sel ou sous la forme d'un extrait naturel, végétal ou de micro-organismes, riche en manganèse.

10. Utilisation selon la revendication précédente, **caractérisée en ce que** le sel de manganèse est choisi parmi les sels organiques ou inorganiques du manganèse.

11. Utilisation selon la revendication 10, **caractérisée en ce que** le sel organique de manganèse est choisi parmi le gluconate de manganèse ou le carbonate de manganèse ou l'acétate de manganèse ou le citrate de manganèse ou l'oléate de manganèse ou l'oxalate de manganèse.

12. Utilisation selon la revendication 10, **caractérisée en ce que** le sel inorganique de manganèse est choisi parmi les sels minéraux comme le chlorure de manganèse ou le borate de manganèse ou le nitrate de manganèse ou le phosphate de manganèse ou le sulfate de manganèse.

13. Procédé de traitement cosmétique des rides et/ou des ridules, consistant à appliquer par voie topique une composition cosmétique comprenant dans un milieu physiologiquement acceptable une quantité efficace de manganèse ou d'au moins l'un de ses sels,

14. Procédé selon la revendication précédente, **caractérisé en ce que** le manganèse est sous forme ionique ou sous la forme d'un sel ou sous la forme d'un extrait naturel, végétal ou de micro-organismes, riche en manganèse.
